# FASCICULE DE BREVET EUROPEEN

(11) **EP 0 712 392 B1**
(45) Date de publication et mention de la délivrance du brevet: **10.06.1998**
(21) Numéro de dépôt: 94924341.4
(22) Date de dépôt: 08.08.1994
(51) Int. Cl.: C07C 409/10, C07C 407/00

(54) **PROCEDE DE PREPARATION D'HYDROPEROXYDE DE CUMENE**
VERFAHREN ZUR HERSTELLUNG VON CUMENHYDROPEROXYD
PROCESS FOR THE PREPARATION OF CUMENE HYDROPEROXIDE

(30) Priorité: 06.08.1993 FR 9309705
(43) Date de publication de la demande: 22.05.1996
(73) Titulaire: RHODIA CHIMIE, 92408 Courbevoie Cédex (FR)
(72) Inventeur: AMADIO, Joao, F-69006 Lyon (FR); LE CORRE, Brice, F-69001 Lyon (FR); CHARRIN, Jean-Jacques, F-69003 Lyon (FR); HOUZARD, Xavier, F-59500 Bron (FR); LAURENT, Philippe, F-38150 Roussillon (FR); NOYERIE, Roland, F-38150 Salaise-sur-Sanne (FR); QUENTON, Yves-Michel Les Terrasses du Rhône, F-69560 Saintes-Colombes (FR)
(74) Mandataire: Dubruc, Philippe
(86) Numéro de dépôt international: FR9400990
(87) Numéro de publication internationale: WO9504717

(56) Documents cités:
- EP-A- 0 032 758
- US-A- 4 262 143
- DATABASE WPI Section Ch, Week 9206, Derwent Publications Ltd., London, GB; Class E14, AN 92-044352 & JP,A,3 287 574 (MITSUI TOATSU CHEM INC) 18 Décembre 1991

## Description

La présente invention se situe dans le domaine de la fabrication du phénol à partir de cumène. Plus particulièrement la présente invention conceme la première étape de ce procédé, à savoir l'obtention d'hydroperoxyde de cumène.

L'obtention de phénol à partir de cumène est un procédé industriel bien connu, effectué en continu et comprenant essentiellement deux étapes.

La première consiste à oxyder, en phase liquide, le cumène en hydroperoxyde de cumène, en utilisant un gaz contenant de l'oxygène. Puis l'hydroperoxyde ainsi obtenu est, dans une seconde étape, décomposé pour donner le phénol et l'acétone.

Avant cette demière étape, il est nécessaire de concentrer l'hydroperoxyde formé. En effet, pour des questions d'efficacité du procédé et de sécurité, la réaction d'oxydation du cumène n'est pas conduite au delà d'une certaine concentration en hydroperoxyde dans le mélange réactionnel. La raison est d'éviter une scission prématurée de l'hydroperoxyde formé, qui peut devenir incontrôlable. Habituellement, la teneur en hydroperoxyde ne dépasse pas 40 % en poids du mélange réactionnel, d'où la nécessité de l'étape de concentration préalable à la scission en phénol.

Durant la première partie du procédé, l'hydroperoxyde de cumène est formé en même temps que divers sous-produits, tels que, notamment, le diméthylphénylcarbinol, le peroxyde de dicumyle, l'acétophénone, de même que le phénol et des acides organiques. La présence de ces derniers composés est reconnue comme néfaste sur la réaction. En effet, les acides favorisent la scission de l'hydroperoxyde en phénol, lui-même étant un inhibiteur de la réaction d'oxydation.

C'est pourquoi de nombreux procédés préconisent de mettre en oeuvre la réaction d'oxydation du cumène en présence de divers additifs, comme notamment des agents neutralisant lesdits composés, agents choisis en général parmi l'hydroxyde, le carbonate d'un métal alcalin ou encore un sel de métal alcalin de l'hydroperoxyde de cumène.

Un premier type de procédé de préparation d'hydroperoxyde de cumène consiste à mettre en oeuvre la réaction en présence d'agent neutralisant dans des quantités de l'ordre de quelques pourcents. Ces procédés présentent cependant des inconvénients dus à la mise en oeuvre de teneurs relativement importantes de tels agents.

D'une part, ces quantités représentent un surcoût de matières premières non négligeables pour le procédé.

D'autre part elles nécessitent l'utilisation d'un matériel supplémentaire avant l'étape de concentration de l'hydroperoxyde de cumène. En effet, il est rappelé que la mise en oeuvre d'additifs de ce type produit des sels de métal alcalin qui doivent être éliminés du mélange réactionnel avant l'étape de concentration, afin d'éviter l'encrassement des appareils utilisés lors de cette étape de concentration. Or cette opération n'est possible qu'en effectuant un ou plusieurs lavages à l'eau du mélange réactionnel. Cependant, une telle méthode nécessite la mise en oeuvre d'une étape de pré concentration du mélange réactionnel lavé, avant l'étape de concentration de l'hydroperoxyde proprement dite.

Un autre moyen pourrait être envisagé pour éliminer les sels de métal alcalin, tel que la filtration du mélange réactionnel. Cependant, les quantités d'agent mis en oeuvre dans les procédés connus rendent impossible l'exploitation d'une telle méthode sur le plan industriel, car les filtres sont très rapidement encrassés par le dépôt des sels et deviennent donc inutilisables. Par ailleurs, cette opération de séparation est relativement délicate car les sels filtrés sont d'une consistance visqueuse, rendant difficiles la filtration même, puis le nettoyage ultérieur des filtres.

Enfin, des problèmes de sécurité peuvent se poser car lesdits sels sont instables et peuvent s'enflammer spontanément lorsqu'ils sont exposés à l'air.

Un second type de procédé de préparation d'hydroperoxyde de cumène consiste à mettre en oeuvre la réaction avec un agent neutralisant employé dans des quantité de l'ordre de quelques centaines de parties par million par rapport au cumène présent dans le milieu. Un tel procédé est notamment décrit dans la demande de brevet japonais JP 3287574. Cependant ce procédé est mis en oeuvre à une pression élevée.

Un autre type de procédé de préparation de l'hydroperoxyde de cumène connu, consiste à effectuer l'oxydation du cumène sans ajout d'un agent neutralisant. Cependant, lorsque, dans le courant de la réaction, l'acidité augmente dans le ou les réacteurs où a lieu ladite réaction, il est préconisé de modifier certaines caractéristiques de fonctionnement du procédé pour retrouver des conditions favorables de réaction. Ainsi, il est possible de baisser la température, tous les autres paramètres de la réaction étant gardés constants, mais avec l'inconvénient de faire diminuer la vitesse d'oxydation et par conséquent la productivité du procédé. Une autre voie possible serait d'augmenter la température afin de conserver une bonne productivité. Mais dans un tel cas, ceci serait effectué au détriment de la sélectivité et vraisemblablement de la sécurité, du fait d'une scission prématurée possible de l'hydroperoxyde de cumène fabriqué.

Le problème du contrôle de la température de réaction de préparation d'hydroperoxyde a notamment été traité dans la demande de brevet européen EP 32758. Ainsi, lorsqu'une dérive de la température est constaté, il est préconisé d'ajouter 0,05 à 20 g d'une substance basique par tonne du mélange réactionnel. En outre, il est indiqué que la température du mélange réactionnel doit être de préférence baissée. Cependant, cette référence ne traite concrètement que le cas de l'obtention d'hydroperoxyde d'éthylbenzène, composé moins réactif que le cumène.

La présente invention a donc pour but de pallier les inconvénients mentionnés des procédés connus de préparation du phénol à partir de cumène, et plus particulièrement de l'étape d'oxydation du cumène. Ainsi, le procédé selon l'invention permet de mettre en oeuvre la réaction d'oxydation précitée avec des vitesses d'oxydations importantes, sans nécessiter pour leur maintien, de se placer, même transitoirement, dans des conditions moins favorables à la productivité. Par ailleurs, le procédé selon l'invention permet de s'affranchir des problèmes d'encrassement des filtres placés avant l'étape de concentration de l'hydroperoxyde formé.

Ces buts et d'autres sont atteints par la présente invention qui conceme donc un procédé de préparation d'hydroperoxyde de cumène, en continu, par oxydation en phase liquide d'un mélange réactionnel comprenant du cumène, en présence d'un gaz contenant de l'oxygène, caractérisé en ce que l'on effectue la réaction d'oxydation en présence d'au moins un agent choisi parmi l'hydroxyde, le carbonate d'un métal alcalin et/ou alcalino-terreux ; ledit agent étant utilisé dans une quantité comprise entre 2 et 10 ppb (exprimé en hydroxyde de sodium) par rapport à la quantité de cumène introduite.

Il a été trouvé d'une façon tout à fait surprenante que des quantités aussi faibles que celles indiquées ci-dessus étaient suffisantes, pour maintenir des conditions d'oxydation exploitables industriellement. A titre de comparaison, les concentrations en agent neutralisant dans le mélange réactionnel étaient considérablement plus élevées selon les techniques antérieures, car habituellement comprises entre 0,1 et quelques pour-cent.

Par ailleurs, du fait de la présence dudit agent, il n'est plus nécessaire, pour maintenir des conditions de fonctionnement stables, de faire varier les paramètres de la réaction mais simplement d'ajuster la quantité en agent neutralisant.

Mais d'autres avantages et caractéristiques apparaîtront plus clairement à la lecture de la description et des exemples qui vont suivre.

Ainsi que cela a été indiqué auparavant, le procédé de préparation du phénol à partir du cumène comprend deux étapes principales, l'oxydation du cumène en hydroperoxyde puis la décomposition dudit peroxyde en un mélange d'acétone et de phénol.

La réaction d'oxydation du cumène est généralement conduite dans un ou plusieurs appareils en série (ou oxydeurs). Plus particulièrement, celle-ci est réalisée dans deux à huit appareils.

Le mélange réactionnel en sortie du demier oxydeur est traité de façon à éliminer les traces de sels de métal alcalin qui s'y trouvent. Toutes les méthodes connues de l'homme du métier pourraient être utilisées. Cependant, d'une façon particulièrement avantageuse, la séparation des sels du mélange réactionnel est effectuée en utilisant la méthode de filtration.

Le mélange réactionnel est ensuite traité afin, d'une part de séparer le cumène n'ayant pas réagi de l'hydroperoxyde de cumène, et d'autre part de concentrer ledit peroxyde jusqu'à obtenir une teneur en ce composé, dans le flux sortant, d'environ 80 à 85 %.

Cette opération peut être réalisée en une ou plusieurs étapes. D'une façon classique, on met en oeuvre le principe de la distillation sous vide, dans une ou plusieurs colonnes de fractionnement.

Le flux d'hydroperoxyde concentré, est ensuite décomposé en phénol et en acétone.

Lors de la première étape du procédé, on effectue l'oxydation du cumène par un gaz contenant de l'oxygène, en une ou plusieurs étapes.

Le cumène introduit dans les oxydeurs est constitué en partie de cumène neuf et en partie de cumène recyclé.

En effet, comme indiqué précédemment, la totalité du cumène introduit n'est pas transformée en hydroperoxyde. Généralement le taux de transformation du cumène est compris entre 20 et 40 %. Aussi, pour des raisons évidentes d'économies, la plupart du temps, il est prévu de recycler dans le procédé le cumène n'ayant pas réagi.

La proportion de cumène recyclé n'est pas critique et l'homme du métier est à même de régler celle-ci.

Quelle que soit la nature du cumène (cumène neuf ou cumène recyclé), celui-ci présente de préférence une pureté d'au moins 99,5 %, et plus particulièrement d'au moins 99,8 %.

Par ailleurs, le cumène est substantiellement exempt d'acide et de phénol.

Ainsi, le cumène n'ayant pas réagi est traité, avant d'être réintroduit dans le procédé, afin de le débarrasser de toutes les impuretés qu'il contient, et plus particulièrement des impuretés acides.

D'une façon classique, on procède à un ou plusieurs cycles de lavages du cumène, chaque étape étant suivie d'une décantation. Dans un premier temps, on effectue un traitement avec une solution aqueuse d'une base choisie notamment parmi les hydroxydes de métal alcalin ou alcalino-terreux. La concentration de ces solutions est comprise entre 10 et 20 %. Puis on effectue une ou plusieurs étapes de lavage/décantation à l'eau du cumène ainsi traité, afin d'éliminer toutes traces de sels de métal alcalin ou alcalino-terreux restantes.

Ces opérations sont en général entreprises à une température se situant entre 25 et 45 °C.

Il est à noter que le procédé selon l'invention ne favorise pas la formation des sous-produits, comme notamment le phénol. Par conséquent, la teneur en matières polluantes, se retrouvant principalement dans les eaux de lavage du cumène est diminuée, facilitant par conséquent l'épuration de celles-ci.

La réaction d'oxydation est effectuée en présence d'un gaz contenant de l'oxygène. On peut utiliser à cette fin toute source d'oxygène, pure ou diluée, telle que l'air, enrichi ou non en oxygène. D'une façon avantageuse, on utilise de l'air comme agent d'oxydation du cumène.

Chacun des oxydeurs est muni de moyen d'introduction de gaz contenant l'oxygène. Habituellement, et pour optimiser la qualité du mélange liquide/gaz, l'introduction d'oxygène est effectuée en pied des oxydeurs, par tout moyen connu de l'homme du métier.

La teneur en oxygène introduite dans le mélange réactionnel est d'au moins 8 % et de préférence d'au moins 20 %.

D'une façon générale et pour des raisons de sécurité, la quantité d'oxygène dans les gaz sortant de chacun des oxydeurs est maintenue inférieure à 6,5 %. Plus particulièrement , la quantité d'oxygène est maintenue entre 2 et 6,5 % et de préférence entre 4,5 et 6,5 %.

La caractéristique essentielle de l'invention réside dans le fait que l'on introduit dans les oxydeurs, un agent choisi parmi l'hydroxyde, le carbonate d'un métal alcalin et/ou alcalino-terreux ; ledit agent étant utilisé dans une quantité considérablement plus faible que celle des procédés connus car comprise entre 2 et 10 ppb (exprimé en hydroxyde de sodium) par rapport à la quantité de cumène introduite.

De préférence, la quantité d'agent introduite est comprise entre 2 et 5 ppb (exprimé en hydroxyde de sodium).

L'agent choisi est plus particulièrement un composé à base d'un métal alcalin, tel que notamment la soude, la potasse, le carbonate de sodium, le carbonate de potassium.

L'agent est mis en contact avec le mélange réactionnel, de préférence sous la forme d'une solution aqueuse. La concentration en agent dans la solution est telle qu'elle permette de respecter la gamme de concentration de 2 à 10 ppb (exprimé en hydroxyde de sodium) dans ledit mélange.

L'introduction de l'agent peut être effectuée en continu ou discontinu, dans l'un ou chacun des oxydeurs. Dans ce demier cas, la quantité totale d'agent dans le mélange réactionnel ne dépasse pas 10 ppb (exprimé en hydroxyde de sodium) par rapport à la quantité de cumène introduite.

L'agent est injecté de telle sorte que le pH du mélange réactionnel reste compris entre 3 et 5.

Le temps de séjour du mélange réactionnel dans chacun des oxydeurs est compris entre 10 à 48 heures.

La température de réaction est comprise entre 70 et 110 °C. De préférence, la température est comprise entre 75 et 90 °C.

Il est à noter que d'une façon classique la température de réaction varie dans les oxydeurs et plus particulièrement qu'elle diminue quand la concentration en hydroperoxyde de cumène augmente.
Cette mesure, connue de l'homme du métier, est nécessaire pour éviter tout risque de décomposition prématurée du peroxyde et conserver un bon rendement.

La réaction d'oxydation peut être effectuée sous pression atmosphérique ou sous une légère surpression. Ainsi, on peut convenablement réaliser l'oxydation à une pression comprise entre 1 et 3 bar absolus.

Le mélange réactionnel après la ou les étapes successives d'oxydation subit une filtration pour éliminer les traces de sels de métal alcalin et/ou alcalino-terreux. D'une façon surprenante, on n'a pas constaté d'encrassement des filtres dû au fait que la réaction d'oxydation du cumène était mise en oeuvre en présence d'un agent alcalin ou alcalino-terreux. Ainsi, le procédé selon l'invention rend possible l'utilisation de filtre à l'échelle industrielle, donc apporte une simplification importante de l'étape de séparation des sels de métal alcalin ou alcalino-terreux du mélange réactionnel.

Le mélange réactionnel ainsi traité est ensuite fractionné et concentré par une distillation sous vide, réalisée en une ou plusieurs étapes.

Le cumène récupéré à l'issue de cette distillation est recyclé vers les oxydeurs, après avoir été purifié selon la méthode indiquée auparavant.

L'hydroperoxyde de cumène est obtenu avec une concentration d'environ 85 %.

La scission de l'hydroperoxyde en phénol et en acétone est ensuite réalisée selon les conditions bien connues de l'homme de l'art.

Des exemples concrets mais non limitatifs du procédé selon l'invention vont maintenant être présentés.

### EXEMPLE:

Dans un réacteur en acier continu, de volume total égal à 8 litres, alimenté en continu par une solution de cumène lavé contenant 0,1 % d'HPOC (hydroperoxyde de cumène), de l'air et une solution aqueuse de soude, est effectué l'étape d'oxydation.

L'impact d'un arrêt de l'alimentation en soude est mis en évidence par la baisse du titre en HPOC, l'augmentation du titre en phénol et acidité : la température doit être augmentée pour conserver la productivité. Cette modification conduit à une dégradation supplémentaire de la masse réactionnelle.

Les quantités de soude injectées pour rétablir un profil d'oxydation normal sont très faibles : de l'ordre de 5 ppb en soude 100 %. Ceci permet une fiabilisation remarquable de l'unité sans dégradation des performances des appareils en aval : concentration et distillation.

Ces résultats sont présentés dans le tableau suivant :

| Intervalle de temps (h) | Cumène (Kg/h) | Soude (mg/h) | T (°C) | HPOC (%) | pH | Phénol (ppm) | Acidité (ppm) | O2 (%) |
|---|---|---|---|---|---|---|---|---|
| 0-62 | 0,1 | 5,10⁻⁴ | 78,2 | 30,1 | 3,6 | 4 | 30 | 6,2 |
| 62-124 | 0,1 | 0 | 80 | 29,5 | - | 4 | 30 | 6,2 |
| 124-186 | 0,1 | 0 | 80,6 | 27,3 | 3,3 | 28 | 55 | 6,5 |
| 186-248 | 0,1 | 5.10⁻⁴ | 78,3 | 27,9 | 3,9 | 8 | 30 | 6,0 |
| 248-310 | 0,1 | 5.10⁻⁴ | 78 | 29 | 3,8 | 4 | 25 | 5,8 |

## Revendications

1. Procédé de préparation d'hydroperoxyde de cumène, en continu, par oxydation en phase liquide d'un mélange réactionnel comprenant du cumène, en présence d'un gaz contenant de l'oxygène, caractérisé en ce que l'on effectue la réaction d'oxydation en présence d'au moins un agent choisi parmi l'hydroxyde, le carbonate d'un métal alcalin et/ou alcalino-terreux ; ledit agent étant utilisé dans une quantité comprise entre 2 et 10 ppb (exprimé en hydroxyde de sodium) par rapport à la quantité de cumène introduite.

2. Procédé selon la revendication 1, caractérisé en ce que l'on utilise une quantité d'agent comprise entre 2 et 5 ppb (exprimé en hydroxyde de sodium) par rapport à la quantité de cumène introduite.

3. Procédé selon l'une des revendications précédentes, caractérisé en ce que l'on utilise un agent choisi parmi la soude, la potasse, le carbonate de sodium, le carbonate de potassium.

4. Procédé selon l'une des revendications précédentes caractérisé en ce que l'on met en oeuvre un flux de cumène d'une pureté d'au moins 99,5 et de préférence d'au moins 99,8.

5. Procédé selon l'une des revendications précédentes caractérisé en ce que l'on met en oeuvre un flux de cumène substantiellement exempt d'acide et de phénol.

6. Procédé selon l'une des revendications précédentes caractérisé en ce que l'on effectue la réaction à une température comprise entre 70 et 110 °C, et de préférence entre 75 et 90°C.

7. Utilisation de l'hydroperoxyde obtenu en mettant en oeuvre le procédé selon l'une quelconque des revendications 1 à 7 pour obtenir du phénol.

## Patentansprüche

1. Verfahren zur kontinuierlichen Herstellung von Cumolhydroperoxid durch Oxidation in flüssiger Phase eines Reaktionsgemischs, das Cumol umfaßt, in Gegenwart eines Sauerstoff enthaltenden Gases, dadurch gekennzeichnet, daß man die Oxidationsreaktion in Gegenwart wenigstens eines Mittels, dasunter dem Hydroxid, dem Carbonat eines Alkali- und/oder Erdalkalimetalls ausgewählt ist, ausführt; wobei besagtes Mittel in einer Menge zwischen 2 und 10 ppb (ausgedrückt als Natriumhydroxid), bezogen auf die eingeführte Cumolmenge, verwendet wird.

2. Verfahren gemäß Anspruch 1, dadurch gekennzeichnet, daß man eine Menge an Mittel zwischen 2 und 5 ppb (ausgedrückt als Natriumhydroxid), bezogen auf die eingeführte Cumolmenge, verwendet.

3. Verfahren gemäß einem der vorangehenden Ansprüche, dadurch gekennzeichnet, daß man ein Mittel verwendet, das unter Natriumhydroxid, Kaliumhydroxid, Natriumcarbonat, Kaliumcarbonat ausgewählt ist.

4. Verfahren gemäß einem der vorangehenden Ansprüche, dadurch gekennzeichnet, daß man einen Durchfluß an Cumol mit einer Reinheit von wenigstens 99,5 und vorzugsweise wenigstens 99,8 einsetzt.

5. Verfahren gemäß einem der vorangehenden Ansprüche, dadurch gekennzeichnet, daß man einen Durchfluß an Cumol, das im wesentlichen frei von Säure und Phenol ist, einsetzt.

6. Verfahren gemäß einem der vorangehenden Ansprüche, dadurch gekennzeichnet, daß man die Reaktion bei einer Temperatur zwischen 70 und 110 °C und vorzugsweise zwischen 75 und 90 °C ausführt.

7. Verwendung des Hydroperoxids, das unter Verwendung des Verfahrens gemäß einem der Ansprüche 1 bis 6 erhalten wurde, zur Herstellung von Phenol.

## Claims

1. A process for continuous preparation of cumene hydroperoxide by liquid phase oxidation of a reaction mixture comprising cumene in the presence of a gas containing oxygen, characterized in that the oxidation reaction is carried out in the presence of at least one agent selected from the hydroxide or carbonate of an alkali and/or alkaline-earth metal; said agent being used in a quantity which is in the range 2 to 10 ppb (expressed as sodium hydroxide) with respect to the quantity of cumene introduced.

2. A process according to claim 1, characterized in that the quantity of agent used is in the range 2 to 5 ppb (expressed as sodium hydroxide) with respect to the quantity of cumene introduced.

3. A process according to claim 1 or claim 2, characterized in that the agent used is selected from sodium hydroxide, potassium hydroxide, sodium carbonate and potassium carbonate.

4. A process according to any one of the preceding claims, characterized in that a stream of cumene is used which has a purity of at least 99.5, preferably at least 99.8.

5. A process according to any one of the preceding claims, characterized in that the cumene stream used is substantially free of acid and phenol.

6. A process according to any one of the preceding claims, characterized in that the reaction is carried out at a temperature which is in the range 70°C to 110°C, preferably in the range 75°C to 90°C.

7. Use of the hydroperoxide obtained using the process according to any one of claims 1 to 7 to produce phenol.
